(19) Europäisches Patentamt
European Patent Office
Office européen des brevets

(11) Publication number: **0 071 286**
**B1**

(12) **EUROPEAN PATENT SPECIFICATION**

(45) Date of publication of patent specification: **28.08.85**

(51) Int. Cl.[4]: **C 07 C 68/00,** C 07 C 69/96, B 01 J 31/28

(21) Application number: **82200851.2**

(22) Date of filing: **07.07.82**

# (54) Process for the preparation of carbonate esters.

(30) Priority: **30.07.81 GB 8123325**

(43) Date of publication of application:
**09.02.83 Bulletin 83/06**

(45) Publication of the grant of the patent:
**28.08.85 Bulletin 85/35**

(84) Designated Contracting States:
**BE DE FR GB IT NL**

(56) References cited:
**EP-A-0 038 984**
**DE-A-2 107 467**
**DE-A-2 110 194**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 071 286 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the preparation of carbonate esters.

Organic carbonates are very versatile reagents in organic synthesis. They may be used for example in the preparation of isocyanates and polycarbonates, and as alkylating agents.

UK Patent Specification No. 1,303,756 discloses that the oxidative carbonylation of an alcohol according to the equation:

$$2\ ROH + CO + 1/2\ O_2 \rightarrow RO-\overset{\overset{O}{\|}}{C}-OR + H_2O$$

where R is an alkyl, aryl or cycloalkyl hydrocarbyl group, may be catalysed by a complexed compound of a metal belonging to Group IB, IIB or VIII of the Periodic Table. Complexes derived from copper salts, such as $Cu_2Cl_2$, and organic bases, for example pyridine, are exemplified as catalysts.

This system has certain disadvantages however, which are discussed in Belgian Patent Specification No. 859,272. Said specification states that the process is improved by the use of a simple metal salt without a complexing agent being present. Copper (I) salts are preferred. Extensive details of this process are also presented in Ind. Eng. Chem. Prod. Res. Dev. *1980* 19 pp. 396—403, where it is concluded that in fact the use of a copper (I) salt is much preferred, since the use of copper (II) salts leads to large quantities of by-products, especially halogen-containing products when using copper (II) halides as catalysts.

The major disadvantage with both of the catalyst systems discussed above is that the presence of water has a marked detrimental effect on both the rate of the reaction and on the selectivity to the desired product. Water is of course produced during the reaction, and thus to maximise reaction rate and selectivity it is necessary to terminate the reaction at low levels of conversion. The detrimental effect of water is discussed in detail on pages 400 and 401 of the literature article cited above.

The Applicants have now found a method of increasing both the rate of the reaction, and the selectivity to the desired product at a given level of conversion of the alcohol starting material.

The invention therefore provides a process for the oxidative carbonylation of an alcohol to produce a carbonate, which comprises reacting together carbon monoxide, oxygen and an alcohol, in the presence of a copper compound and in the presence of a sulphone.

The alcohol reactant may contain one or more —OH groups, the resulting carbonate generally being a polymeric compound if two or more —OH groups are present in the starting material. The or each —OH group is attached to an aliphatic carbon atom; thus the alcohol may be an alkanol or an alkenol in which the alkyl or alkenyl group may be optionally substituted by one or more inert substituents. Inert substituents may for example be selected from halogen atoms and phenyl, alkoxy, alkoxycarbonyl and alkylcarbonyl groups. Preferably the alcohol has up to 20 carbon atoms, especially up to 10 carbon atoms. Most preferably, the alcohol contains only hydrocarbyl moieties except for the —OH group or groups, as for example in methanol, ethanol, benzyl alcohol, allyl alcohol and the various isomeric propanols and butanols.

The copper compound used in the process according to the invention may contain copper in either the (I) or (II) valent state, but preferably a copper (II) compound is added to the reaction mixture. The compound may be a simple salt, for example a salt with an organic acid such as acetic acid or with a mineral acid such as a hydrogen halide or hydrogen cyanide. Alternatively, the compound may be a complex containing both anions and neutral ligands of the type described in UK Patent Specification No. 1,303,756. Such complexes may be preformed and added to the reaction mixture as such, or they may be generated *in situ* by reaction of a copper salt with a suitable donor ligand. It has, surprisingly, been found that especially active catalysts are obtained when a copper salt, especially a copper (I) or, preferably a copper (II), halide, is added to the reaction mixture, there also being present in the reaction mixture a tertiary aliphatic amine, especially a trialkylamine, for example triethylamine. Preferably the number of moles of tertiary aliphatic amine added per gram atom of copper is in the range of from 0.01 to 1, especially 0.05 to 0.9.

The quantity of copper compound present in the reaction mixture is not critical, any amount capable of exerting a catalytic effect being suitable. Quantities in the range of from 0.001 to 10%, especially 0.01 to 5%, calculated as gram atoms of copper per mole of alcohol, are generally suitable. The sulphone may be cyclic or acyclic. Suitable sulphones include those of the general formula

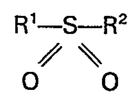

in which each of $R^1$ and $R^2$ independently represents an optionally substituted alkyl group, or $R^1$ and $R^2$ together represent an optionally substituted alkylene group. Optional substituents may be any moieties inert under the reaction conditions, for example halogen atoms, alkoxy groups, phenyl groups and phenoxy groups. An alkyl group $R^1$ or $R^2$ preferably has up to 12, especially up to 6, carbon atoms. An alkylene group represented by $R^1$ and $R^2$ together preferably has 4, 5 or 6 carbon atoms in the chain, and up to 6 carbon atoms in any alkyl side-chain. Preferably $R^1$ and $R^2$ represent unsubstituted alkyl groups, or $R^1$ and $R^2$ together represent an unsubstituted alkylene group.

Specific examples of suitable acyclic sulphones are dimethyl, diethyl, dipropyl, dibutyl, methyl ethyl, and methyl butyl sulphone. Specific examples of cyclic sulphones are sulpholane, 2-methylsulpholane, 3-methylsulpholane, 3-butylsulpholane, 3-isopropylsulpholane, and 2-methyl-4-butylsulpholane. The use of sulpholane is especially preferred.

The quantity of sulphone used is not critical, and may vary over a wide range. For example, the molar ratio of sulphone to alcohol reactant may be in the range of from 0.05:1 to 20:1, especially 0.25:1 to 10:1. If the sulphone is a liquid at the reaction temperature, it may conveniently be used in solvent amounts; it is often most convenient to use the minimum quantity of liquid sulphone required to produce a homogeneous reaction mixture.

If desired, an inert solvent may be present in the reaction mixture, for example a hydrocarbon or halogenated hydrocarbon such as pentane, toluene or carbon tetrachloride; an ester such as ethyl acetate; a ketone such as acetone; or an ether such as diethyl ether or tetrahydrofuran. In order to facilitate work-up of the reaction mixture, which contains water generated as a co-product along with the carbonate, it is often convenient to use an inert solvent which forms an azeotrope either with the water or with the carbonate. In this way the water and the carbonate can be separated readily.

The presence of water in the reaction mixture can be tolerated in the process according to the invention. Some water may even be present at the beginning of the reaction, and the presence of the amounts of water normally found in commercial forms of components of the reaction mixture presents no problem.

The reaction is preferably carried out in the presence of halide ion. This may originate from the copper salt, for example if a copper halide is used, or it may come from another source of halide; for example, an alkali metal halide, an alkaline earth metal halide or a hydrogen halide may be added to the reaction mixture.

The reaction is also preferably carried out in the presence of a base. Organic bases such as aliphatic tertiary amines as discussed above, or alkali or alkaline earth metal carboxylates, are suitable. Suitable inorganic bases include alkali and alkaline earth metal carbonates. Preferably the number of moles of base added per gram atom of copper is in the range of from 0.01 to 1, especially 0.05 to 0.9.

The process according to the invention is preferably carried out at a temperature in the range of from 50 to 150°C, especially 70 to 125°C. Pressures in the range of from 10 to 100 bars, especially 30 to 80 bars, are preferred. Higher pressures may of course be used, but are in general uneconomical.

The ratio of carbon monoxide to oxygen used is not critical for the reaction, the main consideration being that of safety—i.e. to ensure that the $CO/O_2$ ratio is outside the explosive limits. It may for example be convenient to conduct the reaction under a high partial pressure of carbon monoxide, and to meter in oxygen throughout the course of the reaction.

Preferably the concentration of oxygen in the reaction vessel is maintained throughout the reaction within the range of about 3% to about 8% by volume of the gas phase. Inert gases, for example nitrogen, may of course be present; it may for example be convenient to meter air into the reaction mixture as the source of oxygen.

The following Examples illustrate the invention.

Examples 1 to 9

All these experiments were carried out by the following general method. A Hastelloy C (Trade Mark) 300 ml magnet-driven autoclave was charged with the stated quantities of alcohol, sulpholane, catalyst, and any additional components. The sulpholane was normal water-containing commercial grade. The autoclave was then pressurised with 50 bars carbon monoxide (except Example 1, 30 bars) and the temperature was raised to the desired level. Air at 4 bars was then introduced periodically, and the internal oxygen level was monitored by a leak-stream to ensure that the oxygen concentration remained at less than 8% by volume of the gas cap, and greater than 3%. Thus the pressure within the autoclave remained roughly constant, the CO removed during the course of the reaction being replaced by the nitrogen present in the air introduced. After the stated reaction time, the contents of the autoclave were analyzed by gas-liquid chromatography.

The results of the experiments are given in the following Table. In all cases, the methanol was converted to dimethylcarbonate, with virtually no by-product formation; traces (less than 0.5%) of dimethoxymethane, methyl chloride and methyl chloroformate were the only by-products observed. In Examples 2, 4 and 6, almost no carbon monoxide remained in the autoclave after the reaction time was over. Example 5 shows that the presence of quite large quantities of water at the beginning of the reaction leads to some reduction in the rate of the reaction but no reduction in selectivity to the desired product.

TABLE OF RESULTS

| Example No. | Catalyst (mmol) | Methanol (mls) | Supholane (mls) | Other components present | Reaction temperature (°C) | Reaction time (hours) | Conversion of methanol of products (% molar) |
|---|---|---|---|---|---|---|---|
| 1 | $CuCl_2 . 2H_2O$ (6) | 25 | 25 | $N(C_2H_5)_3$ (7 mmol) | 90 | 1 | 10 |
| 2 | $CuCl_2 . 2H_2O$ (12) | 25 | 25 | $N(C_2H_5)_3$ (4 mmol) | 120 | 1.5 | 31 |
| 3 | $CuCl_2 . 2H_2O$ (12) | 10 | 40 | — | 125 | 4 | 20 |
| 4 | $CuCl_2 . 2H_2O$ (12) | 10 | 40 | $N(C_2H_5)_3$ (4 mmol) | 125 | 3 | 48 |
| 5 | $CuCl_2 . 2H_2O$ (12) | 10 | 40 | $N(C_2H_5)_3$ (4 mmol) $H_2O$ (5 mls) | 92 | 2 | 17 |
| 6 | $CuCl_2 . 2H_2O$ (12) | 5 | 45 | $N(C_2H_5)_3$ (4 mmol) | 120 | 4 | 62 |
| 7 | CuCl (12) | 10 | 40 | — | 90 | 3 | 9 |
| 8 | CuCl (12) | 10 | 40 | $N(C_2H_5)_3$ (4 mmol) | 90 | 3 | 15 |
| 9 | $Cu(CH_3CO_2)_2$ (12) | 25 | 25 | $N(C_2H_5)_3$ (4 mmol) HCl (2 mls of 37% aqueous solution) | 100 | 3 | 20 |

Example 10 (comparison)

Example 1 was repeated exactly except that the 25 mls sulpholane were replaced by an additional 25 mls methanol, and the reaction time was 2.5 hours. After this time, less than 1% of the methanol had been converted to dimethylcarbonate.

**Claims**

1. A process for the oxidative carbonylation of an aliphatic alcohol to produce a carbonate, which comprises reacting together carbon monoxide, oxygen and an alcohol, in the presence of a copper compound and in the presence of a sulphone.

2. A process as claimed in claim 1, in which the copper compound contains copper in the (II) valent state.

3. A process as claimed in either claim 1 or claim 2, in which the sulphone has the general formula

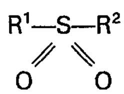

in which each of $R^1$ and $R^2$ independently represents an optionally substituted alkyl group, or $R^1$ and $R^2$ together represent an unsubstituted alkylene group.

4. A process as claimed in claim 3, in which $R^1$ and $R^2$ represent unsubstituted alkyl groups, or $R^1$ and $R^2$ together represent an unsubstituted alkylene group.

5. A process as claimed in claim 4, in which the sulphone is sulpholane.

6. A process as claimed in any one of claims 1 to 5, carried out in the presence of halide ions.

7. A process as claimed in any one of claims 1 to 6, carried out in the presence of a base.

8. A process as claimed in claim 7, carried out in the presence of a tertiary aliphatic amine.

9. A process as claimed in claim 8, carried out in the presence of a trialkylamine.

10. A process as claimed in any one of claims 7 to 9, in which the number of moles of base added per gram atom of copper is in the range of from 0.05 to 0.9.

**Patentansprüche**

1. Ein Verfahren zur oxidativen Carbonylierung eines aliphatischen Alkohols zwecks Erzeugung eines Carbonats, welches das Miteinander-Umsetzen von Kohlenmonoxid, Sauerstoff und einem Alkohol in Gegenwart einer Kupferverbindung und in Gegenwart eines Sulfons umfaßt.

2. Ein Verfahren wie in Anspruch 1, beansprucht, in welchem die Kupferverbindung Kupfer im zweiwertigen Zustand enthält.

3. Ein Verfahren wie sowohl in Anspruch 1 als auch in Anspruch 2 beansprucht, in welchem das Sulfon die nachstehende Formel

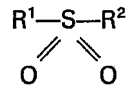

aufweist, in welcher jedes $R^1$ und $R^2$ unabhängig voneinander eine gegebenenfalls substituierte Alkylgruppe darstellt oder $R^1$ und $R^2$ zusammen eine unsubstituierte Alkylengruppe bilden.

4. Ein Verfahren wie in Anspruch 3 beansprucht, in welchem $R^1$ und $R^2$ unsubstituierte Alkylgruppen darstellen oder $R^1$ und $R^2$ zusammen eine unsubstituierte Alkylengruppe bilden.

5. Ein Verfahren wie in Anspruch 4 beansprucht, in welchem das Sulfon die Verbindung Sulfolan ist.

6. Ein Verfahren wie in irgendeinem der Ansprüche 1 bis 5 beansprucht, durchgeführt in Gegenwart von Halogenionen.

7. Ein Verfahren wie in irgendeinem der Ansprüche 1 bis 6 beansprucht, durchgeführt in Gegenwart einer Base.

8. Ein Verfahren wie in Anspruch 7 beansprucht, durchgeführt in Gegenwart eines tertiären aliphatischen Amins.

9. Ein Verfahren wie in Anspruch 8 beansprucht, durchgeführt in Gegenwart eines Trialkylamins.

10. Ein Verfahren wie in irgendeinem der Ansprüche 7 bis 9 beansprucht, in welchem die Molzahl der zugesetzten Base je Grammatom Kupfer im Bereich von 0,05 bis 0,9 liegt.

**Revendications**

1. Procédé de carbonylation par oxydation d'un alcool aliphatique pour produire un carbonate, dans lequel on fait réagir ensemble du monoxyde de carbone, de l'oxygéne et un alcool, en présence d'un composé de cuivre et en présence d'une sulfone.

2. Procédé selon la revendication 1, où le composé de cuivre contient du cuivre à l'état de valence (II).

3. Procédé selon la revendication 1 ou la revendication 2, où la sulfone à la formule générale

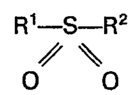

ou chacun des radicaux $R^1$ et $R^2$ represente indépendamment un groupe alcoyle éventuellement substitué, ou $R^1$ et $R^2$ representent ensemble un groupe alcoylène non substitué.

4. Procédé selon la revendication 3, où $R^1$ et $R^2$ représentent des groupes alcoyle non substitués, ou bien où $R^1$ et $R^2$ représentent ensemble un groupe alcoylène non substitué.

5. Procédé selon la revendication 4, où la sulfone est le sulfolane.

6. Procédé selon l'une quelconque des revendications 1 à 5, conduit en présence d'ions halogénure.

7. Procédé selon l'une quelconque des revendications 1 à 6, conduit en présence d'une base.

8. Procédé selon la revendication 7, conduit en présence d'une amine aliphatique tertiaire.

9. Procédé selon la revendication 8, conduit en présence d'une trialcoylamine.

10. Procédé selon l'une quelconque des revendications 7 à 9, où le nombre de moles de base ajoute par atome-gramme de cuivre est dans un intervalle allant de 0,05 à 0,9.